Europäisches Patentamt

European Patent Office

Office européen des brevets

(11) Publication number: **0 105 065**
**A1**

(12) **EUROPEAN PATENT APPLICATION**

(21) Application number: **82305307.9**

(22) Date of filing: **05.10.82**

(51) Int. Cl.³: **A 61 K 9/24**

(43) Date of publication of application: **11.04.84**
**Bulletin 84/15**

(84) Designated Contracting States: **AT BE CH DE FR GB IT LI LU NL SE**

(71) Applicant: **Mathur, Kishan Narain, 24 Tennyson Avenue, Wanstead London E11 2QN (GB)**

(72) Inventor: **Mathur, Kishan Narain, 24 Tennyson Avenue, Wanstead London E11 2QN (GB)**

(74) Representative: **Frankland, Nigel H. et al, FORRESTER & BOEHMERT Widenmayerstrasse 4/I, D-8000 München 22 (DE)**

(54) **A pharmaceutical composition for pain relief.**

(57) A pharmaceutical composition comprising a tranquillizer or sedative, a first analgesic and a second analgesic, the composition being such that, on oral administration, the tranquillizer or sedative is absorbed first, the first analgesic is absorbed second and the second analgesic is absorbed last.

A particularly preferred embodiment comprises an outer layer of diazepam, an intermediate layer of parcetamol and an inner enteric-coated core of aspirin. The diazepam acts first to calm the patient, the paracetamol is then absorbed in the stomach for swift analgesic action and, after a delay, the aspirin is absorbed in the intestine for a secondary analgesic action.

THIS INVENTION relates to a pharmaceutical composition for administration to humans or animals suffering from pain.

According to one aspect of this invention, there is provided a pharmaceutical composition comprising a tranquillizer or sedative, a first analgesic and a second analgesic, the composition being such that, on oral administration, the tranquillizer or sedative is absorbed first, the first analgesic is absorbed second and the second analgesic is absorbed last.

Preferably, a tranquillizer is used instead of a sedative, suitable compounds being diazepam and chlordiazepoxide, although it is to be appreciated that many other tranquillizers could be used.

The first and second analgesics may be the same as one another although, preferably, they are not. In one particularly preferred embodiment, the first analgesic is paracetamol and the second analgesic is aspirin. Clearly, many other analgesic compounds, such as codeine, could be used as either the first or second analgesic.

Preferably, in order to delay absorption of the second analgesic, the second analgesic is coated with an enteric layer such as cellacephate.

According to a second aspect of the invention, there is provided a tablet comprising a composition according to the first aspect of the invention.

Preferably, the tranquillizer or sedative forms an outer layer of the tablet, the first analgesic forms an intermediate layer of the tablet and the second analgesic forms a core of the tablet.

Conveniently, the central core of the second analgesic has an enteric coating.

So that the present invention may be more readily understood and so that further features thereof may be appreciated, preferred embodiments of the invention will now be discussed by way of example.

A preferred embodiment of a tablet in accordance with the invention comprises a central core of 600 mg aspirin with an enteric coating, surrounded by an intermediate layer of 500 mg paracetamol and an outer layer of 1 mg diazepam. The tablet is intended for 6 hourly oral administration in cases of acute and severe pain, although the frequency of administration can be increased in particularly severe cases to a frequency of 6 to 8 tablets in a 24 hour period. The tablet should preferably be taken after meals.

Tablets in accordance with the invention have been found to be useful in reducing or eliminating pain and the perception of pain in patients suffering from rheumatic and musculo-skeletal disorders, toothache, headache, lower back pain, lumbago, slipped disc and dysmenorrhoea.

It has been found that the first clinical effect of the tablet is a reduction of the patient's anxiety and tension due to the immediate absorption of the tranquilliser or sedative. In many patients of a nervous disposition, their anxiety caused by the painful condition can trouble them more than the pain itself and it has been found that the immediate action of the tranquillizer or sedative acts both to reduce this anxiety and also, as a result of the reduction in the anxiety, to reduce the patient's perception of the painful condition itself. Clearly, a tranquilliser will be preferable to a sedative in this context, although in some circumstances it may be desirable for a sedative to be present, either instead of or in conjunction with the tranquillizer. In addition, the diazepam, which is the preferred tranquillizer, will act as a muscle relaxant, which can be of direct assistance in reducing the pain associated with certain muscular disorders.

The second clinical effect of the tablet arises from the swift absorption of the first analgesic. In the specific example given above, the first analgesic is paracetamol, but many other common analgesic compounds would be suitable, for example aspirin or codeine. Absorption of the first analgesic takes place in the stomach and, for this reason, occurs rapidly after ingestion of the tablet.

The specific tablets described above contains a unitary core of enteric-coated aspirin, the aspirin constituting the second analgesic of the invention. By virtue of the enteric coating, the central core of the tablet will pass through the stomach without being absorbed and will only be absorbed when the integrity of the enteric coating is lost in the intestine. Thus, there is a delay between the absorption of the first analgesic and the absorption of the second analgesic, resulting from the delay in the passage of the tablet through the gut from the stomach to the intestine. This delay is increased by administering the tablets after meals.

Instead of having a single core with a single enteric coating, it is possible for the second analgesic (namely aspirin in the preferred embodiment described above) to be present in a plurality of discrete regions, each having a respective enteric coating. In this way, it is possible for the second analgesic to be dispersed throughout the region occupied by the first analgesic. Although the formulation of such a tablet may be more time-consuming than the preparation of the specific example described above, such a modified tablet allows the thickness of the enteric coating surrounding the regions of second analgesic to be varied so that the period of time during which the second analgesic is absorbed can be extended to be equal to, in the extreme, the time taken for the remains of the tablet to pass completely through the intestine and rectum.

By delaying absorption of the aspirin until the tablet enters the intestine, it has been found possible to reduce the gastric bleeding associated with aspirin.

The immediate absorption of the tranquilliser or sedative in the specific example described above is achieved by locating the diazepam in an outer layer surrounding the entire tablet and enclosing the intermediate paracetamol layer. However, it is possible to create a slight delay between the absorption of the diazepam and the absorption of the paracetamol by other means, for example by enclosing the paracetamol in a physical barrier which is degraded in the stomach. Thus, one such modified tablet might comprise generally planar layers of diazepam, paracetamol and aspirin, the paracetamol being enclosed within, for example, a sugar coating which would be rapidly destroyed in the stomach, and the aspirin having the enteric coating referred to above.

Administration of tablets in accordance with the invention would be contra-indicated in the following conditions:

Renal and hepatic disorders, allergies, pregnancy, breast-feeding, bleeding disorders, pulmonary insufficiency, and peptic ulceration. In addition, the tablet should not be administered to children, the ingestion of alcohol should be avoided during administration of the tablets, and continuous long term use is not recommended.

It is to be appreciated that the invention encompasses any novel feature or combination of features described herein.

0105065

CLAIMS:

1.    A pharmaceutical composition comprising a tranquillizer or sedative, a first analgesic and a second analgesic, the composition being such that, on oral administration, the tranquillizer or sedative is absorbed first, the first analgesic is absorbed second and the second analgesic is absorbed last.

2.    A composition according to claim 1 wherein a tranquillizer is used instead of a sedative.

3.    A composition according to claim 2 wherein the tranquillizer is diazepam.

4.    A composition according to any one of the preceding claims wherein the two analgesics are not the same as one another.

5.    A composition according to any one of the preceding claims wherein the first analgesic is paracetamol.

6.    A composition according to any one of the preceding claims wherein the second analgesic is aspirin.

7.    A composition according to any one of the preceding claims wherein, in order to delay absorption of the second analgesic, the second analgesic is coated with an enteric layer.

8.    A tablet or pill comprising a composition according to any one of the preceding claims.

9.    A tablet or pill according to claim 8 wherein  the tranquillizer or sedative forms an outer layer of the tablet, the first analgesic forms an intermediate layer of the tablet and the second analgesic forms a core of the tablet.

10.    A tablet or pill according to claim 9 wherein the central core of the second analgesic has an enteric coating.

0105065

11.  A composition according to any one of claims 1 to 7 for use in relieving pain.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | GB-A-1 276 089 (PFIZER) * Page 1, column 1, line 29 - column 2, line 84; page 2, column 1, lines 21-40; example 1; claims 1,10 * | 1,4-11 | A 61 K 9/24 |
| Y | CHEMICAL ABSTRACTS, vol. 71, no. 14, 6th October 1969, page 226, no. 64124s, Columbus, Ohio, USA A. DAL POZZO: "Analytical study of some common therapeutic associations. II. Analytical methods for mixtures of tranquilizers, analgesics, and local anesthetics" & BOLL. CHIM. FARM. 1969, 108(4), 230-4 * Abstract * | 1-3,11 | |
| Y | CHEMICAL ABSTRACTS, vol. 92, no. 6, 11th February 1980, page 332, no. 47185d, Columbus, Ohio, USA I.D. BRADBROOK et al.: "Enhanced absorption of salicylate from safapryn-Co tablets" & BR. J. CLIN. PHARMACOL. 1979, 8(4), 371-2 * Abstract * | 1,4-11 | TECHNICAL FIELDS SEARCHED (Int. Cl. 3) A 61 K 9/00 |
| Y | CHEMICAL ABSTRACTS, vol. 93, no. 22, 1st December 1980, page 366, no. 210261g, Columbus, Ohio, USA & JP - A - 80 47618 (LION DENTIFRICE CO., LTD.) 04-04-1980 * Abstract * | 1,4-6, 8,11 | |

The present search report has been drawn up for all claims

| Place of search THE HAGUE | Date of completion of the search 03-06-1983 | Examiner BERTE M.J. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82